Europäisches Patentamt

European Patent Office

Office européen des brevets

(11) Publication number : **0 608 989 A2**

(12) **EUROPEAN PATENT APPLICATION**

(21) Application number : **94300279.0**

(22) Date of filing : **14.01.94**

(51) Int. Cl.$^5$ : **A61K 7/02, A61K 7/48**

(30) Priority : **23.01.93 GB 9301297**

(43) Date of publication of application :
**03.08.94 Bulletin 94/31**

(84) Designated Contracting States :
**AT BE CH DE DK ES FR GB GR IE IT LI LU NL PT SE**

(71) Applicant : **THE PROCTER & GAMBLE COMPANY**
**One Procter & Gamble Plaza**
**Cincinnati Ohio 45202 (US)**

(72) Inventor : **Langlois, Anne**
**36 St. Olaves Close**
**Staines, Middlesex TW18 2LH (GB)**

(74) Representative : **Brooks, Maxim Courtney et al**
**Procter & Gamble Limited**
**Whitley Road**
**Longbenton**
**Newcastle-upon-Tyne NE12 9TS (GB)**

(54) **Cosmetic make-up compositions.**

(57)    A make-up composition comprising humectant, primary and auxiliary emulsifier, stabilizing agent, pigment, and a pre-emulsified silicone/polyglycerylmethacrylate lubricant.
     The make-up composition exhibits superior moisturisation and skin feel together with reduced tackiness and excellent skin coverage.

EP 0 608 989 A2

Jouve, 18, rue Saint-Denis, 75001 PARIS

## Field of the Invention

The present invention relates to cosmetic make-up compositions and more particularly, to pigmented foundation make-up compositions and blushers.

## Background of the Invention

A foundation composition can be applied to the face and other parts of the body to even skin tone and texture and to hide pores, imperfections, fine lines and the like. A foundation composition is also applied to moisturize the skin, to balance the oil level of the skin and to provide protection against the adverse effects of sunlight, wind and the harsh environment.

Make-up compositions are generally available in the form of liquid or cream suspensions, emulsions, pressed powders or anhydrous oil and wax compositions. Make-up in the form of a gel is also available and has some advantages over other forms.

US Patent No. 3,444,291 discloses a method of filling and camouflaging skin cavities by applying a composition which includes 65 to 75 parts by weight of a microcrystalline wax and about 25 to 35 parts of a mineral oil. The composition includes a colourant, preferably a coal tar dye, for example, D &C Red No. 17, which matches the colour of the user's skin.

A spreadable, flowable and greaseless cosmetic cover-up composition is taught in US Patent No 4,486,405. That composition is characterized by the presence of a first and a second alkoxylated surfactant present in substantially the same concentration.

US Patent No. 4,804,532 recites a facial cosmetic powder which utilizes crystalline silica in much lower concentration than that employed in the then prior art compositions. This powder, used as a blush or a facial coating, is said to be effective in hiding skin wrinkles, lines and pores. The composition is a mixture of a colour phase and a diluent phase. The colour phase is formed by blending crystalline silica with colourants. The resultant colour phase is mixed with the diluent phase, essentially formed from nacreous materials such as talc and mica, to form the composition.

The use of a foundation composition which has a significantly high concentration of nacreous material is taught in US Patent No. 3,978,207. This foundation, a pressed powder composition, is characterized by the presence of a nacreous material such as mica and a binder oil which provides a frosted pearl effect, that is, a lustrous look. The colour of this foundation is provided by the nacreous material.

US Patent No. 4,659,562 discloses a cosmetic make-up composition which includes, as a binding agent therefore, an intimate mixture of from 5 to 95 weight percent of a mixture of finely divided silica and about 5 to 95 weight percent of finely divided polyethylene fibres. The composition is recited to maintain its uniformity over the areas of the skin to which it is applied. That is, it is said to be "creaseproof". The composition of the '562 patent includes colourant in admixture with nacreous agents.

Nakamura et al., Preprints of the XIVth I.F.S.C.C. Congress, Barcelona, 1986, Vol. I, 51-63 (1986) describes a novel make-up composition utilizing spherical silica and polydimethyl siloxane. This combination is recited to provide a foundation which reduces wrinkle visibility to a greater extent than makeup foundations with which it was compared. This reduction in wrinkle visibility is caused by optical blurring enhanced by the novel use of spherical silica and polydimethyl siloxane.

Polymeric materials which have the capability to stabilize pigmented cosmetics have long been available; however, the stabilizing agents for a practicable make-up composition must not interfere to any significant extent with other components of the formulation.

In the past, humectants such as water-soluble polyglycerylmethacrylate lubricants and glycerine have been incorporated into skin and hair compositions for use as gelling and moisturisation agents. These compositions have provided improvements in moisturisation, absorption, skin feel, residue and skin care characteristics compared with conventional cosmetic cream and lotion compositions. They have, however, also suffered negatives such as tackiness and shine, especially when large concentrations of humectant are employed. These problems can be further aggravated in pigmented make-up compositions which by their nature tend to be somewhat tacky in use.

Many thickeners, both hydrophilic and hydrophobic, are available but are not suitable at the critical concentration needed for system stabilization or gelation due to the presence of electrolytes, interference with pigment dispersion, or failure to provide an aesthetically pleasing end product due to tackiness and heavy skin feel.

It is accordingly one object of this invention to provide a make-up composition in the form of a stabilized emulsion or gel.

It is also an object of the invention to provide a pigmented make-up composition which exhibits improved moisturisation, skin-feel application and wear characteristics.

It is a further object of the invention to provide a pigmented make-up composition which exhibits reduced tackiness and improved skin coverage and wear characteristics.

## Summary of the Invention

In accordance with one aspect of this invention, there is provided a make-up composition comprising:

a) from about 1% to about 30% by weight of a pre-emulsified silicone/polyglycerylmethacrylate lubricant;

b) from about 1% to about 30% by weight of a water-soluble humectant or mixture of humectants;

c) from about 2% to about 15% by weight of an emulsifier;

d) from about 0.1% to about 3 % of a stabilizing agent selected from cellulosic polymers, synthetic biopolymer gums, thixotropic clays, and mixtures thereof;

e) from about 2% to about 25 % by weight of pigment; and,

f) from about 20% to about 50% by weight of water.

The compositions of the present invention provide improved moisturisation and skin feel, without consumer negatives such as tackiness. Furthermore, the compositions herein give excellent skin coverage and wear characteristics. In preferred compositions, the make-up takes the form of a gel (i.e. has a non-zero yield point).

The compositions of this aspect of the present invention are stable, are easily applied to the skin, and retain their gel-like properties for an adequate time needed for smooth application and are non-greasy. The presence of the stabilizing agents helps create a moisture balance that is defined by the combination of water-soluble and oil-soluble moisturizers in the composition. Further, the make-up can withstand prolonged exposure to low and high temperatures when protected in a package, and yet retain its gel-like properties.

All levels and ratios are by weight of total composition, unless otherwise indicated. Chain length and degrees of ethoxylation are also specified on a weight average basis.

## Detailed Description of the Invention

The make-up composition of the present invention comprises a pre-emulsified silicone/polyglycerylmethacrylate lubricant, a water-soluble humectant or mixture of humectants, emulsifier, a stabilizing agent, and a pigment.

A first essential component is a pre-emulsified silicone/polyglycerylmethacrylate lubricant. The pre-emulsified silicone/polyglycerylmethacrylate lubricants which can be used in the compositions of this invention are available under the trademark, Lubrasil (RTM) from the Guardian Chemical Corporation, 230 Marcus Blvd., Hauppage, N.Y. 11787. In general, Lubrasil (RTM) can be described as a mergence of silicone oil and polyglycerylmethacrylate lubricant in which the silicone oil is microemulsified using high energy to form a complex. Lubrasil also comprises propylene glycol and Polysorbate 20.

Suitable polyglycerylmethacrylate lubricants herein include those having a viscosity (neat) of at least about 50,000 mPa.s, preferably at least about 80,000 mPa.s, and mixtures thereof (viscosities being measured with a Brookfield RVT Viscometer at 20°C).

The polyglycerylmethacrylate lubricants which can be used in making the compositions of this invention are available under the trademark Lubrajel (RTM) from Guardian Chemical Corporation, 230 Marcus Blvd., Hauppage, N.Y. 11787. In general, Lubrajels can be described as hydrates or clathrates which are formed by the reaction of sodium glycerate with a methacrylic acid polymer. Thereafter, the hydrate or clathrate is stabilized with a small amount of propylene glycol, followed by controlled hydration of the resulting product. Lubrajels are marketed in a number of grades of varying glycerate : polymer ratio and viscosity. Suitable Lubrajels include TW, Lubrajel CG and Lubrajel MS, Lubrajel WA, Lubrajel DV and so-called Lubrajel Oil.

The pre-emulsified silicone/polyglycerylmethacrylate lubricant is present in the compositions herein at a level of from about 1% to about 30%, preferably from about 1% to about 20% by weight of composition.

The water-soluble humectant or mixture of humectants herein is present in an amount of from about 1% to about 30% by weight, suitable humectants are selected from glycerine and polyglycerylmethacrylate lubricant having a viscosity at 25°C of 300,000 to 1,100,000 cps; a specific gravity at 25°C of 1 to 1.2 g/ml, a pH of 5.0 to 5.5; a bound water content of 33 to 58%; and, a free water content from 5 to 20%.

Other suitable humectants include sorbitol, panthenols, propylene glycol, butylene glycol, hexylene glycol, alkoxylated glucose derivatives, such as Glucam (RTM) E-20, hexanetriol, and glucose ethers, and mixtures thereof. Urea is also suitably added as a humectant.

Polyglycerylmethacrylate lubricants having the desired properties are marketed by Guardian Chemical Corporation under the trademark "Lubrajel". The "Lubrajels" identified as "Lubrajel DV", "Lubrajel MS", and "Lubrajel CG" are preferred in the present invention. The gelling agents sold under these trademarks contain about 1% propylene glycol. In preferred embodiments, however, the composition contains less than about 8 %, preferably from about 5 % of polyglycerylmethacrylate lubricant which has not been pre-emulsified with silicone, this being preferred from the viewpoint of optimum skin feel and application characteristics.

Preferably, each humectant is present in an amount of from about 5% to about 15% by weight.

There is also present in the compositions herein from about 1% to about 15%, preferably from about 4% to about 12.5% by weight of an emulsifier. The emulsifier preferably comprises a primary emulsifier and an auxiliary emulsifier.

The primary emulsifier preferably is the reaction product of an amine and a fatty acid and most preferably is the reaction product of an amine with a saturated fatty acid having a straight chain length from about 16 to about 18 carbon atoms, such as, for example, stearic or palmitic acid. The concentration of the primary emulsifier in the composition may be from about 2% to about 5% by weight of the total composition, and preferably is present in an amount of from about 2.5 to about 3 % by weight.

In the preferred method of carrying out the present invention, the amine and the fatty acid are mixed together to form the primary emulsifier during one of the process steps for making the make-up composition.

Examples of suitable auxiliary emulsifying agents are natural waxes such as, for example, beeswax; polyethylene glycol derivatives of sterols; glycerol esters of fatty acids such as, for example, stearic acid and palmitic acid; lecithin; alkylphosphates of diethanolamine; and, hydroxylated lecithin. Suitable alkylphosphates of diethanolamine are $C_{12}$-$C_{18}$ and preferably $C_{16}$ alkyls. The total concentration of the auxiliary emulsifier preferably is from about 1% by weight to about 7.5% by weight, and most preferably is in the range from about 3 to about 4% by weight of the total composition.

Suitable stabilizing agents include cellulosic polymers, synthetic biopolymer gums, and thixotropic clays. The preferred cellulosic polymer is hydroxyethyl cellulose; the preferred synthetic biopolymer gum is xanthan gum, and the preferred thixotropic clay is magnesium aluminium silicate. The concentration of the stabilizing agent is usually from about 0.1 to 1.0% by weight of the total composition, although higher levels up to eg., 2 or 3 % can be used if desired.

The pigment used in the compositions of the present invention can be inorganic and/or organic. Also included within the term pigment are materials having a low colour or lustre such as matte finishing agents, and also light scattering agents. Examples of suitable pigments are iron oxides, acylglutamate iron oxides, ultramarine blue, D&C dyes, carmine, and mixtures thereof. Depending upon the type of make-up composition, whether foundation or blusher, a mixture of pigments will normally be used.

The foundation composition can also include at least one matte finishing agent. The function of the matte finishing agent is to hide skin defects and reduce shine. Such cosmetically acceptable inorganic agents, i.e., those included in the CTFA Cosmetic Ingredient Dictionary, Third Ed., as silica, hydrated silica, mica, talc, polyethylene, titanium dioxide, bentonite, hectorite, kaolin, chalk, diatomaceous earth, attapulgite and the like may be utilized. Of particular usefulness as a matte finishing agent is low lustre pigment such as titanated mica (mica coated with titanium dioxide) coated with barium sulfate. Of the inorganic components useful as a matte finishing agent low lustre pigment, talc, polyethylene, hydrated silica, kaolin, titanium dioxide and mixtures thereof are particularly preferred. Materials suitable for use herein as light-scattering agents can be generally described as spherical shaped inorganic materials having a particle size of up to about 100 microns, preferably from about 5 to about 50 microns, for example spherical silica particles.

The total concentration of the pigment may be from about 2 to about 25 % by weight and is preferably from 7 to about 11% by weight of the total composition, the exact concentration being dependent to some extent upon the specific mixture of pigments selected for use in a foundation make-up or blusher. The preferred compositions contain from about 2% to about 20% by weight of titanium dioxide and most preferably about 6% by weight of titanium dioxide.

The preferred pigments for use herein from the viewpoint of moisturisation are treated pigments. The pigments can be treated with compounds such as amino acids, silicones, lecithin and ester oils, preferably amino acids.

The balance of the composition of the present invention is deionized water which is typically present in an amount of about 20-50% by weight.

Also preferably present in the make-up compositions herein is an emollient. Emollients suitable for the compositions of the present invention include hydroxy acid esters; fatty acid esters; polyoxyalkylene ethers; polyalkylene glycols; linear silicones; cyclomethicones and other volatile silicones; lanolin, lanolin derivatives such as lanolin alcohol, ethoxylated lanolin alcohol, hydroxylated lanolin, acetylated lanolin and cholesterol; long chain hydrocarbons such as squalane, mineral oil and petrolatum; straight and branched chain diesters and triesters; esters derived from C8 alcohols; and mixtures thereof.

The cyclomethicones are low molecular weight polydimethylsiloxanes exemplified by the formula:

$$( (CCH_3)_2SiO)_x$$

4

where x is either 4 or 5. The fatty acid esters are selected from carboxylic acids containing about 8 to 22 carbon atoms. Examples of suitable hydroxy acid esters include citrates and maleates. The long chain hydrocarbons generally have about 20 to 60 carbon atoms.

Preferred emollients are selected from cetearyl isononanoate isopropyl palmitate, isopropyl isostearate, cetyl octanoate, cetyl acetate, trioctyl citrate, PEG isoceteth-3 acetate, dioctyl maleate, propylene glycol dicaprylate/dicaprate, caprylic/ capric triglyceride, cyclomethicones, mineral oil, dimethicone, PPG-20 methylglucose ether, and lanolin alcohol, and mixtures thereof. These emollients may be used independently or in mixtures and may be present in the composition of the present invention in an amount from about 1% to about 30% by weight, and preferably are present in an amount from about 5% to about 20% by weight of the total composition.

The composition may also contain additional materials such as, for example, fragrances, fillers such as nylon, sun-screens, preservatives, proteins, antioxidants, and chelating agents.

Another optional component of the make-up composition is one or more ultraviolet absorbing agents. Ultraviolet absorbing agents, often described as sunscreening agents, can be present in a concentration in the range of between about 1% and about 12% by weight, based on the total weight of composition. Preferably, the UV absorbing agents constitute between about 2% and 8% by weight. More preferably, the UV absorbing agents are present in composition in a concentration range of between about 4% and about 6% by weight. Of the ultraviolet absorbing agents suitable for use herein, benzophenone-3, octyl dimethyl PABA (Padimate O) and mixtures thereof are particularly preferred.

A chelating agent may also be incorporated in the make-up composition. A chelating agent is preferably present in the composition in a concentration in the range of between about 0.02% to about 0.10% by weight, based on the total weight of the composition. Preferably, the chelating agent is present in a concentration in the range of between about 0.03% and about 0.07% by weight, based on the total weight of the composition. Among the chelating agents that may be included in the composition is trisodium EDTA.

Another optional but preferred component of the make-up composition is one or more preservatives. The preservative concentration in the make-up composition, based on the total weight of that composition, is in the range of between about 0.2% and about 0.8% by weight, preferably between about 0.4% and about 0.6% by weight. Suitable preservatives for use herein include diazolidinyl urea, methyl paraben and ethyl paraben and mixtures thereof.

The make-up compositions of the present invention can be in the form of foundations, blushers, concealers, compact powders, and the like, preferably as foundations and blushers.

The following Table is provided to illustrate make-up compositions of the present invention:

| Example | I | II | III | IV | V | VI |
|---|---|---|---|---|---|---|
| | Wt % | Wt % | Wt % | Wt % | Wt % | Wt % |
| **A.** | | | | | | |
| Squalane | -- | -- | -- | -- | -- | 2.50 |
| Octyl Methoxycinnamate | -- | -- | -- | -- | -- | 5.00 |
| Benzophenone-3 | -- | -- | -- | -- | -- | 2.00 |
| Hydroxylated Lecithin | -- | -- | -- | -- | -- | 2.50 |
| Lecithin | -- | -- | -- | -- | -- | 0.40 |
| Vitamin E Linoleate | -- | -- | -- | -- | -- | 0.50 |
| Cetyl Alcohol | -- | -- | -- | -- | -- | 1.00 |
| Dimethicone | -- | -- | -- | -- | -- | 0.50 |
| Cetyl Phosphate | -- | -- | -- | -- | -- | 0.50 |
| Dioctyl Maleate | 3.03 | 5.50 | 7.70 | 5.50 | 5.50 | -- |
| Cetearylisononanoate | 2.89 | 5.25 | 7.35 | 5.25 | 5.25 | -- |
| Propylene Glycol Dicaprylate/Dicaprate | 2.89 | 5.25 | 7.35 | 5.25 | 5.25 | 9.50 |
| Cyclomethicone | 1.65 | 0.5 | 4.20 | 0.5 | 3.00 | 10.00 |
| Glyceryl Monostearate | 1.50 | 2.25 | 3.15 | 2.25 | 2.25 | 1.25 |
| Cyclomethicone /Dimethiconol 85:15 | -- | 2.5 | -- | 2.5 | -- | -- |
| Stearic Acid | 1.50 | 1.50 | 2.10 | -- | 2.00 | 2.5 |
| Palmitic Acid | -- | -- | -- | 1.50 | -- | -- |
| Mineral Oil | 0.83 | 1.50 | 2.10 | 1.50 | 1.50 | -- |
| Beeswax | 0.50 | 0.50 | 0.50 | 0.50 | 0.50 | 0.75 |
| Phenoxethol | 0.21 | 0.21 | 0.21 | 0.21 | 0.21 | 0.21 |
| Propylparabens | 0.10 | 0.10 | 0.10 | 0.10 | 0.10 | 0.10 |
| Parabens | 0.09 | -- | -- | -- | -- | -- |
| BHA | -- | -- | -- | -- | -- | 0.10 |
| **B.** | | | | | | |
| Titanium Dioxide | 6.00 | 6.00 | 6.00 | 8.00 | 2.00 | 10.00 |
| Talc | 2.10 | 2.10 | 2.10 | 2.10 | 2.16 | 3.00 |
| Yellow Iron Oxide | 0.34 | 0.34 | 0.34 | 0.34 | 0.06 | 0.81 |

| Example | I Wt % | II Wt % | III Wt % | IV Wt % | V Wt % | VI Wt % |
|---|---|---|---|---|---|---|
| Red Iron Oxide | 0.46 | 0.46 | 0.46 | 0.46 | -- | 0.60 |
| Black Iron Oxide | 0.08 | 0.08 | 0.08 | 0.10 | 0.02 | 0.14 |
| Ultramarine Blue | 0.02 | 0.02 | 0.02 | -- | -- | 0.05 |
| Titanated Micas | -- | -- | -- | -- | 6.16 | 2.40 |
| D&C Red #30 | -- | -- | -- | -- | 0.68 | -- |
| Nylon | -- | -- | -- | -- | -- | 0.50 |

C.

| | I | II | III | IV | V | VI |
|---|---|---|---|---|---|---|
| Deionized Water | - - - - - - - - - - - To 100- - - - - - - - - - - | | | | | |
| Triethanolamine | 0.75 | 0.75 | 0.90 | 0.75 | 1.00 | 1.00 |
| Methylparaben | 0.1 | 0.1 | 0.1 | 0.1 | 0.1 | 0.1 |
| Trisodium EDTA | 0.01 | 0.01 | 0.01 | 0.01 | 0.01 | 0.05 |
| Allantoin | -- | -- | -- | -- | -- | 0.10 |
| Phenoxethol | 0.49 | 0.49 | 0.49 | 0.49 | 0.49 | 0.49 |
| Parabens | 0.21 | 0.21 | 0.21 | 0.21 | 0.21 | 0.21 |
| Propylene Glycol | 3.00 | 3.00 | 3.00 | 3.00 | 3.00 | -- |
| Hydroxyethylcellulose | 0.20 | 0.20 | 0.20 | 0.20 | 0.20 | -- |

E.

| | I | II | III | IV | V | VI |
|---|---|---|---|---|---|---|
| Deionized Water | 4.75 | 4.75 | 4.75 | 4.75 | 4.75 | -- |
| Magnesium Aluminium Silicate | 0.25 | 0.25 | 0.25 | 0.25 | 0.25 | -- |

F.

| | I | II | III | IV | V | VI |
|---|---|---|---|---|---|---|
| Glycerine | 10.00 | 10.00 | 10.00 | 5.00 | 10.00 | 10.00 |
| Propylene Glycol | 2.00 | 2.00 | 2.00 | 2.00 | 2.00 | 6.00 |
| Xanthan Gum | 0.08 | 0.08 | 0.08 | 0.08 | 0.08 | 0.20 |

EP 0 608 989 A2

| Example | I Wt % | II Wt % | III Wt % | IV Wt % | V Wt % | VI Wt % |
|---|---|---|---|---|---|---|
| **G.** | | | | | | |
| PEG-10 Soya Sterol | 0.75 | 0.75 | 1.15 | 0.75 | 1.10 | 2.00 |
| **H.** | | | | | | |
| Deionized Water | 1.00 | 1.00 | 1.00 | 1.00 | 1.00 | 2.00 |
| Diazolidinyl Urea | 0.25 | 0.25 | 0.25 | 0.25 | 0.25 | 0.30 |
| **I.** | | | | | | |
| Essential Oils | 0.20 | 0.20 | 0.20 | 0.20 | 0.15 | -- |
| Perfume Oil | 0.20 | 0.20 | 0.20 | 0.20 | 0.15 | 0.25 |
| Vitamin A Palmitate | -- | -- | -- | -- | -- | 0.05 |
| **J.** | | | | | | |
| Aloe Vera Gel | 3.00 | 3.00 | 3.00 | 3.00 | -- | 3.00 |
| Chamomile Extract | -- | -- | -- | -- | -- | 0.10 |
| **K.** | | | | | | |
| Lubrasil | 17.00 | 16.00 | 14.00 | 15.00 | 18.00 | 12.00 |

The various components listed in the Table have been segregated into groups, the constituents of each group being mixed together before being added to members of the remaining groups in accordance with the procedures set forth below.

In the first step, the mixture of the components of oil phase A is heated to a temperature of about 72°C, and additional cyclomethicone fluid is added to compensate for its loss through evaporation. When phase A has melted and has reached a temperature of about 72°C, the pigments of phase B are mixed into the heated mixture.

The components of aqueous phase C are heated together to a temperature of about 50-55°C while stirring to dissolve the methyl paraben. The propylene glycol/hydroxyethylcellulose slurry of phase D is added to heated aqueous phase C. The magnesium aluminium silicate dispersion of phase E is prehydrated for at least 30 minutes at 50°C and is then added to the aqueous phase C and thoroughly dispersed therein.

The propylene glycol (or glycerin)/xanthan gum slurry of phase F is added to heated aqueous phase C at 60-65°C and dispersed therein. The resulting aqueous phase containing the components of phases C, D, E and F is then heated to about 70°C ± 2°C.

The PEG-10 soya sterol of phase G is added to the blend of phases A and B at 70°C. The resulting oil phase of components A, B and G is then added to the water phase of components C, D, E and F and agitated

8

via stirring to emulsify the mixture at a temperature of about 70°C.

The resulting mixture is cooled down. At about 55°C, water is added to compensate for loss through evaporation, and the deionized water-diazolidinyl urea mixture of phase H is added to phases A-G at about 45°C. At about 35°C, the Lubrasil of phase K is added to the resulting batch. The resulting mixture is agitated with high speed mixing until a gel-like consistency is obtained, generally within about 1-2 minutes. At 40°C, the perfume oil and essential oils of phase I are added and at 37°C, the aloe vera gel of phase J is added.

The resulting make-up composition is ready for packaging.

The make-up compositions of the Examples exhibit improved moisturisation and skin-feel benefits, reduced tack and improved skin coverage.

## Claims

1. A make-up composition comprising:
   a) from about 1% to about 30% by weight of pre-emulsified silicone/polyglycerylmethacrylate lubricant;
   b) from about 1% to about 30% by weight of a water-soluble humectant or mixture of humectants;
   c) from about 1% to about 15% by weight of an emulsifier;
   d) from about 0.1% to about 3 % of a stabilizing agent selected from cellulosic polymers, synthetic biopolymer gums, thixotropic clays, and mixtures thereof;
   e) from about 2% to about 25 % by weight of pigment; and,
   f) from about 20% to about 50% water.

2. A make-up composition according to Claim 1 wherein the water-soluble humectant or mixture of humectants is selected from glycerine and polyglycerylmethacrylate lubricants having a viscosity of from about 300,000 to 1,100,000 cps at 25°C, and mixtures thereof.

3. A make-up composition according to Claim 1 or 2 wherein the emulsifier comprises:
   a) from about 2% to about 5 % by weight of a primary emulsifier comprising the reaction product of an amine and a fatty acid; and,
   b) from about 1% to about 7.5% by weight of an auxiliary emulsifier.

4. A make-up composition according to any of Claims 1 to 3 additionally comprising from about 1% to about 30% by weight of an emollient selected from fatty acid esters, polyoxyalkylene ethers, polyalkylene glycols, linear silicones, cyclomethicones, long chain hydrocarbons, lanolin, lanolin derivatives, hydroxy acid esters, straight and branched chain diesters and triesters, esters derived from C8 alcohols, and mixtures thereof.

5. A make-up composition according to Claim 4 wherein the lanolin derivative is selected from lanolin alcohol, ethoxylated lanolin alcohol, hydroxylated lanolin, acetylated lanolin and cholesterol.

6. The make-up compositions according to Claim 3 wherein the auxiliary emulsifier is selected from natural wax, glycerol ester of a fatty acid, alkylphosphate of diethanolamine, lecithin, hydroxylated lecithin, polyethylene glycol derivative of a sterol, and mixtures thereof.

7. The make-up composition according to Claim 6 wherein the amount of said auxiliary emulsifier is about 3 to about 4% by weight of the total composition.

8. A make-up composition according any of Claims 1 to 7 wherein the pre-emulsified silicone/polyglycerylmethacrylate lubricant is present in an amount of from about 1% to about 20% by weight.

9. A make-up composition according to Claim 3 wherein the primary emulsifier is the reaction product of triethanolamine and stearic acid.

10. A make-up composition according to Claim 3 wherein the primary emulsifier is the reaction product of triethanolamine and palmitic acid.

11. A make-up composition according to Claim 3 wherein the primary emulsifier is present in a amount of from about 2.5% to about 3.0% by weight.

12. A make-up composition according to Claim 4 wherein the emollient is selected from isopropyl palmitate, isopropyl isostearate, dioctyl maleate, propylene glycol dicaprylate/propylene glycol dicaprate, caprylic triglyceride/capric triglyceride,, cyclomethicone, dimethicone, squalane, mineral oil, cetearylisononanoate, lanolin alcohol and mixtures thereof;

13. A make-up composition according to any of Claims 1 to 12 wherein the stabilizing agent is selected from hydroxyethyl cellulose, cellulose gum, xanthan gum, magnesium aluminium silicate, and mixtures thereof.

14. A make-up composition according to any of Claims 1 to 13 additionally comprising one or more ultraviolet absorbing agents.